# EUROPÄISCHE PATENTANMELDUNG

(11) **EP 2 907 529 A1**
(43) Veröffentlichungstag der Anmeldung: **19.08.2015**
(21) Anmeldenummer: 15155647.9
(22) Anmeldetag: 18.02.2015
(51) Int. Cl.: A61L 2/20, A61L 2/24, B29C 49/42

(54) **Verfahren und Vorrichtung zum Sterilisieren von Kunststoffvorformlingen mit temperiertem Gehäuse**

(30) Priorität: 18.02.2014 DE 102014102031
(71) Anmelder: Krones AG, 93073 Neutraubling (DE)
(72) Erfinder: Loy, Michael, 93073 Neutraubling (DE); Söllner, Jürgen, 93073 Neutraubling (DE); Schiessl, Hans-Peter, 93073 Neutraubling (DE)
(74) Vertreter: Bittner, Bernhard

(57) **Zusammenfassung**

Verfahren zum Sterilisieren von Kunststoffvorformlingen, wobei die Kunststoffvorformlinge entlang eines vorbestimmten Transportpfads von einer Transporteinrichtung (2) transportiert werden und wenigstens zeitweise während dieses Transports durch Beaufschlagung mit einem fließfähigen Sterilisationsmittel sterilisiert werden, wobei die Kunststoffvorformlinge wenigstens zeitweise innerhalb eines Gehäuses (4) transportiert werden. Erfindungsgemäß wird wenigstens ein Abschnitt des Gehäuses (4) wenigstens zeitweise und/oder während des Sterilisationsvorgangs durch eine von dem Sterilisationsmittel unabhängige Wärmequelle erwärmt. Alternativ oder zusätzlich ist zumindest ein Abschnitt des Gehäuses (4) gegenüber der Umgebung der Vorrichtung thermisch isoliert.

## Beschreibung

Die vorliegende Erfindung bezieht sich auf ein Verfahren und eine Vorrichtung zum Sterilisieren von Kunststoffvorformlingen. Im Bereich der getränkeherstellenden Industrie ist es allgemein bekannt, dass zunächst Kunststoffvorformlinge zur Verfügung gestellt werden, diese anschließend in einem Ofen erwärmt und mittels beispielsweise Blasformmaschinen zu Kunststoffbehältnissen expandiert werden.

Bei zahlreichen Anwendungen bzw. abzufüllenden Getränken ist es dabei vorteilhaft oder erforderlich, dass die Behältnisse selbst sterilisiert werden. Insbesondere die Haltbarkeit von sensiblen Füllprodukten, insbesondere in PET-Flaschen, kann entscheidend verbessert werden, wenn vor dem Füllprozess die Anzahl der Keime in den Behältnissen deutlich reduziert wird. Dazu sind im Bereich der Fülltechnik verschiedene Nass- und Trockenaseptik-Methoden bekannt. Ein grundsätzlicher Nachteil besteht bei diesen Methoden darin, dass diese aufgrund der hohen Behältervolumina einen hohen Verbrauch an Sterilisationsmedium (wie beispielsweise Peressigsäure oder Wasserstoffperoxid) benötigen. Neuere Maschinenkonzepte basieren daher darauf, dass die Anzahl der Keime bereits vor dem Blasen der Behältnisse in einer Blasformmaschine reduziert wird. Mit anderen Worten werden hier die Kunststoffvorformlinge bzw. "Preforms" sterilisiert. Zu diesem Zweck durchläuft der Kunststoffvorformling einen Behandlungsbereich, in dem die Entkeimung insbesondere durch gasförmige oder flüssige Sterilisationsmittel oder auch durch Bestrahlung (etwa mit UV- oder Elektronenstrahlung) erzielt wird.

Wird zur Entkeimung ein gasförmiges Sterilisationsmittel wie beispielsweise H₂O₂ verwendet, so muss dieser Aggregatszustand während des gesamten Entkeimungsprozesses in dem Behandlungsbereich aufrechterhalten werden. Nur auf diese Weise kann eine konstant hohe Konzentration des Sterilisationsmediums und dadurch ein robuster Entkeimungsprozess mit konstant hoher Entkeimungsrate sichergestellt werden.

Ein Problem bei dieser Art der Sterilisation stellt jedoch insbesondere auch die Gehäusewandung eines Behandlungsraumes dar. Damit die Konzentration des Sterilisationsmittels konstant bleibt, soll nämlich verhindert werden, dass an keiner Stelle die Temperatur unter den Taupunkt des Gemisches fällt.

Im Stand der Technik wird üblicherweise diese Temperierung der Wandung auch direkt durch das Sterilisationsmedium bzw. dessen Temperatur erreicht. Dies bedeutet, dass das dem Sterilisator zugeführte Prozessgas so hoch eingestellt wird, dass eine entsprechende Temperierung des Behandlungsbereiches bzw. auch der Wandungen erreicht werden kann. Dies führt jedoch zu einem unnötig hohen Energieverbrauch und zu einer hohen thermischen Belastung der mechanischen Bauteile.

Der vorliegenden Erfindung liegt daher die Aufgabe zugrunde, den Energieverbrauch derartiger Vorrichtungen zu reduzieren. Daneben soll bevorzugt auch eine allzu hohe Belastung der einzelnen Bauteile derartiger Vorrichtungen vermieden werden. Diese Aufgaben werden erfindungsgemäß durch ein Verfahren und eine Vorrichtung nach den unabhängigen Ansprüchen erreicht. Vorteilhafte Ausführungsformen und Weiterbildungen sind Gegenstand der Unteransprüche.

Bei einem erfindungsgemäßen Verfahren zum Sterilisieren von Kunststoffvorformlingen werden die Kunststoffvorformlinge entlang eines vorbestimmten Transportpfades von einer Transporteinrichtung transportiert und wenigstens zeitweise während dieses Transports durch Beaufschlagung mit einem fließfähigen Sterilisationsmittel sterilisiert. Dabei werden die Kunststoffvorformlinge wenigstens zeitweise innerhalb eines Gehäuses transportiert.

Erfindungsgemäß wird wenigstens ein Abschnitt des Gehäuses wenigstens zeitweise vor und/oder während des Sterilisationsvorganges durch eine von dem Sterilisationsmittel unabhängige Wärmequelle erwärmt. Alternativ oder zusätzlich ist es neben der Erwärmung möglich, dass wenigstens ein Sterilisationsbereich, in dem die Kunststoffvorformlinge sterilisiert werden, gegenüber einer Umgebung thermisch isoliert wird. Bevorzugt findet diese Erwärmung zumindest auch zeitweise und bevorzugt ständig während des Sterilisationsvorgangs statt. Unter dem Sterilisationsvorgang wird dabei insbesondere das Beaufschlagen der Kunststoffvorformlinge mit dem Sterilisationsmedium verstanden. Im Rahmen der Beaufschlagung kann das Sterilisationsmedium aktiv (etwa mit Sprüheinheiten) auf die Kunststoffvorformlinge aufgebracht werden oder auch in diese eingefüllt werden. Es wäre jedoch auch möglich, dass die Kunststoffvorformlinge lediglich durch eine Atmosphäre transportiert werden, welche das Sterilisationsmittel enthält. Der zusätzlichen oder alternativen thermischen Isolierung liegt der Gedanke zugrunde, dass es beispielsweise möglich wäre, diesen Raum einmal zu erwärmen und dieses Temperaturniveau durch die thermische Isolierung zu erhalten. Auch wäre es denkbar, dass durch diese thermische Isolierung ein bestimmtes ausreichendes Temperaturniveau leichter aufrecht erhalten wird. So könnten beispielsweise die Begrenzungswandungen des Gehäuses aus einem thermisch isolierenden Material aufgebaut sein. So könnte beispielsweise für Wandungen des Gehäuses ein Material mit mehreren Schichten verwendet werden.

Bevorzugt werden jedoch die Kunststoffvorformlinge wenigstens zeitweise mittels Beaufschlagungseinrichtung wie etwa Düsenelementen mit dem Sterilisationsmittel beaufschlagt. Bevorzugt werden dabei diese Beaufschlagungseinrichtungen wenigstens zeitweise mit den zu sterilisierenden Kunststoffvorformlingen mitbewegt. Bevorzugt wird im Rahmen des Sterilisationsvorgangs zumindest auch eine Innenoberfläche der Kunststoffvorformlinge mit dem Sterilisationsmittel beaufschlagt. Dabei wäre es möglich dass derartige Beaufschlagungseinrichtung wenigstens teilweise in die Kunststoffvorformlinge eingeführt werden. Alternativ oder zusätzlich kann jedoch auch eine Außenoberfläche der Kunststoffvorformlinge mit dem Sterilisationsmittel beaufschlagt werden.

Es wäre jedoch auch möglich, dass bereits vor dem eigentlichen Sterilisationsvorgangs mit einer Erwärmung begonnen wird, beispielsweise im Rahmen eines Vorwärmens. Neben den (Wand)abschnitten des Gehäuses können noch andere Bereiche der Vorrichtung erwärmt werden, wie beispielsweise Träger für ein Sterilisationsrad, Führungen, Behandlungselemente und dergleichen. Diese Erwärmung könnte dann mittels der thermischen Isolierung leichter aufrecht erhalten werden.

Wie bereits oben erwähnt, wird die Konzentration des Sterilisationsmediums durch ein festes Verhältnis zwischen der Menge an Sterilisationsmittel, beispielsweise H₂O₂, und einer Menge an heißer Luft bestimmt. Die heiße Luft dient dabei als Trägermedium für das Sterilisationsmittel. Je höher die Konzentration an Sterilisationsmittel, wie beispielsweise H₂O₂, in der Luft ist, desto höher liegt auch der Taupunkt dieses Gemisches. Damit die Konzentration im Behandlungsbereich konstant bleibt, sollte an keiner Stelle die lokale Temperatur unter den Taupunkt des Gemisches fallen.

Dann nämlich würde das Sterilisationsmittel, wie H₂O₂, auskondensieren und dadurch die Konzentration sinken. Bei Vorrichtungen aus dem Stand der Technik ist bedingt durch die schlechte Wärmeübertragung zwischen dem Prozessgas und der Einhausung des Behandlungsbereiches eine Lufttemperatur erforderlich, die wesentlich höher ist als die, welche für den Transport von H₂O₂ erforderlich wäre.

Die Erfindung schlägt nunmehr vor, die genannten Gehäusebereiche und insbesondere Gehäusewandungen unabhängig von dem Sterilisationsmittel zu erwärmen bzw. eine separate Erwärmung des Behandlungsbereiches vorzusehen und/oder zumindest ein bestimmtes Temperaturniveau durch die thermische Isolierung aufrecht zu erhalten. Durch diese funktionelle Trennung der beiden Aufgaben des Erwärmens des Behandlungsbereiches und des gasförmigen Transportes des Wasserstoffperoxids bzw. des Sterilisationsmittels oder durch die thermische Isolierung können auch beide Funktionen einzeln optimiert werden.

Diese Vorgehensweise erlaubt es, dass die Temperatur in dem Prozessgas, d. h. insbesondere das fließfähige Sterilisationsmittel, auf das zum Transport des Sterilisationsagens, wie beispielsweise Wasserstoffperoxid, nötige Niveau abgesenkt werden kann. Auf diese Weise sinkt auch die thermische Belastung der einzelnen Bauteile der Vorrichtung. In diesem Zusammenhang wird darauf hingewiesen, dass es sich bei dem fließfähigen Sterilisationsmittel sowohl um das eigentliche Agens selbst, wie beispielsweise Wasserstoffperoxid, handeln kann, als auch eine Mischung zwischen heißer Luft und Agens. Vorteilhaft wird das fließfähige Sterilisationsmittel durch einen Mischvorgang hergestellt.

Insbesondere handelt es sich dabei um einen Mischvorgang zwischen einem (ebenfalls fließfähigen) Sterilisationsagens, wie insbesondere - aber nicht ausschließlich - H₂O₂, und einem weiteren fließfähigen, insbesondere gasfähigen, Medium, und insbesondere Luft, und besonders bevorzugt Steril-Luft.

Durch die erfindungsgemäße Vorgehensweise kann weiterhin die Einhausung des Behandlungsbereichs separat mit Erwärmungstechnologien temperiert werden. Diese können dabei einen wesentlich besseren Wärmeübergang aufweisen. Auf diese Weise wird das Heizverfahren effektiver und auch der Wirkungsgrad steigt insgesamt.

Bei einer weiteren vorteilhaften Ausführungsform handelt es sich bei dem Sterilisationsmittel um ein gas- oder dampfförmiges Sterilisationsmittel. Vorteilhaft handelt es sich bei dem Sterilisationsmittel um Wasserstoffperoxid bzw. eine Wasserstoffperoxid-Mischung. Vorteilhaft wird das Sterilisationsmittel, und insbesondere das Wasserstoffperoxid, auf einer Temperatur gehalten oder eine Temperatur gebracht, welche gerade zum gasförmigen Transport des Sterilisationsmittels bzw. des Agens genügt.

Die Erfindung wird hier unter Bezugnahme auf Kunststoffvorformlinge beschrieben und beansprucht. Es wird jedoch darauf hingewiesen, dass die Erfindung auf die Sterilisation anderer Behältnisse, wie beispielsweise Kunststoffflaschen, Glasflaschen, und dergleichen, Anwendung finden kann. Daneben kann die Erfindung auch auf die Sterilisation von Behältnisverschlüssen Anwendung finden.

Bei einer bevorzugten Ausgestaltung des Verfahrens wird der besagte Abschnitt mit einem weiteren fließfähigen Medium beaufschlagt. Dabei kann es sich insbesondere um erwärmte oder heiße Luft handeln, die auf diesen Abschnitt aufgebracht wird. Dabei ist es möglich, dass die Beaufschlagung der Wand bereits zu einem Zeitpunkt beginnt, zu dem der Sterilisationsprozess noch nicht begonnen hat. Damit wird vorteilhaft eine Vortemperierung des Sterilisationsbereiches bzw. des Gehäuses vorgenommen. Bevorzugt handelt es sich bei dem Abschnitt des Gehäuses um wenigstens eine Gehäusewand (bzw. eine Deckelwand oder eine Bodenwand). Es wäre jedoch auch möglich, dass mehrere oder alle Gehäusewandungen erwärmt werden. Auch können andere Elemente des Gehäuses erwärmt werden, wie etwa in das Gehäuse eingebaute Fenster. Auch ist es möglich, dass diese Gehäusewandungen Öffnungen aufweisen, etwa um Behältnisse zu- oder abzuführen.

Auch wäre es denkbar, mehrere technische Prinzipien vor Erwärmung der Abschnitte einzusetzen, wie etwa eine elektrische Erwärmung von Fenstern und eine Erwärmung anderer Wandabschnitte mittels erwärmter Luft

Alternativ könnten jedoch auch elektrische Heizeinrichtungen Anwendung finden. Auch wäre eine Erwärmung mit einem flüssigen und/oder dampfförmigen Medium denkbar.

So wäre es beispielsweise möglich, dass separate Heizeinrichtungen in dem Behandlungsbereich installiert werden, wie beispielsweise beheizte Fenster. Vorteilhaft wird das erwärmte fließfähige Medium einer weiteren Vorrichtung zum Behandeln von Kunststoffvorformlingen entnommen. Wie bereits oben erwähnt, ist es bei Vorrichtungen aus dem Stand der Technik bekannt, dass diese mehrere - üblicherweise hintereinander geschaltete - Anlagenteile aufweisen. Einige diese Anlagenteile, wie insbesondere die Öfen, mit denen die Kunststoffvorformlinge erwärmt werden, geben dabei in großem Maße Abwärme ab.

So wäre es, wie oben erwähnt z. B. möglich, die Abwärme des Heizofens einer Streckblasmaschine zu nutzen, wobei hierzu bevorzugt das erwärmte Medium über entsprechende Rohrleitungen oder Leitbleche zu der Sterilisationseinrichtung gelangen kann. Die Abwärme dieses Heizofens weist ein nutzbares Temperaturniveau von etwa 30 °C bis 60 °C auf. Würde man dieses Temperaturniveau auf den Behandlungsbereich übertragen, so kann die Temperatur des Sterilisationsmediums auf ein Niveau von etwa 90 °C bis 110 °C abgesenkt werden. Damit wird hier die warme Abluft, bevorzugt die des Heizofens, zu der Sterilisationseinrichtung zum Heizen zugeführt und kann insbesondere zur Verbesserung der Umfeldhygiene gereinigt werden. Neben des hier erwähnten Ofens zum Erwärmen der Kunststoffvorformlinge können jedoch auch andere Maschinen ausgenutzt bzw. zu deren Abwärme verwendet werden. So kann beispielsweise die Abwärme eines Hochleistungskompressors einer Blasmaschine ebenfalls und/oder zusätzlich verwendet werden.

Daneben können auch mehrere weitere Anlagenteile gemeinsam bzw. gleichzeitig als Quelle für ein erwärmtes Medium dienen.

In einer weiteren vorteilhaften Ausführungsform werden die Kunststoffvorformlinge innerhalb eines Reinraums sterilisiert. Dieser Reinraum kann dabei bevorzugt durch das oben erwähnte Gehäuse abgeschlossen sein bzw. werden. Dabei können Schleuseneinrichtungen vorgesehen sein, um dem Heizraum die Kunststoffvorformlinge zuzuführen.

Bei einer weiteren vorteilhaften Ausführungsform wird ein Profil des Gehäuses an ein Profil des Transportpfads der Kunststoffvorformlinge angepasst.

Auf diese Weise kann eine Flächenminimierung des zu erwärmenden Wandabschnitts erreicht werden. Bei einem zu erwärmenden Wandabschnitt kann es sich auch um einen Boden- oder Deckelabschnitt handeln.

Allgemein wird bevorzugt das erwärmte fließfähige Medium gereinigt, bevor es zu dem erwärmendem Wandabschnitt gelangt. Auf diese Weise wird dem Umstand Rechnung getragen, dass die Sterilisation üblicherweise innerhalb eines Reinraums stattfindet. Zum Reinigen dieses Medium können beispielsweise Schwebstofffilter (z. B. H 14) eingesetzt werden. Dieses Reinigen führt auch zu einer deutlich niedrigeren Konzentration an Mikroorganismen, insbesondere im Bereich des Einlaufs der Sterilisationseinrichtung. Bevorzugt wird jedoch zum Erwärmen des Abschnitts eine Wärmequelle verwendet, welche sich in der näheren Maschinenumgebung der Sterilisationseinrichtung befindet.

Bei einer weiteren vorteilhaften Ausführungsform wird wenigstens ein Sterilisationsbereich, in dem die Kunststoffvorformlinge sterilisiert werden, gegenüber einer Umgebung thermisch isoliert. Insbesondere werden die oben besagten Wandabschnitte, welche zumindest diesen Sterilisationsbereich begrenzen, thermisch gegenüber einer Umgebung isoliert. Dies kann beispielsweise in einfacher Weise dadurch erfolgen, dass die zu erwärmende Wand gegenüber einer weiteren äußeren Wand mittels einer Luftschicht abgetrennt wird.

Um nämlich die eingesetzte Wärme besser im Behandlungsbereich halten zu können, ist es zusätzlich zu der Erwärmung bzw. Beheizung sinnvoll, den gesamten Bereich nach außen hin zu isolieren. Neben den geringeren Wärmeverlusten liegt ein weiterer Vorteil darin, dass der Einfluss der Umgebungstemperatur auf die Sterilisationsbedingungen minimiert wird. Auf diese Weise ist der gesamte Sterilisationsprozess auch gegenüber Temperaturschwankungen, beispielweise saisonalen Temperaturschwankungen, unabhängig.

Die vorliegende Erfindung ist weiterhin auf eine Vorrichtung zum Sterilisieren von Kunststoffvorformlingen gerichtet, welche eine Transporteinrichtung aufweist, welche die Kunststoffvorformlinge entlang eines vorgegebenen Transportpfads transportiert. Weiterhin weist die Vorrichtung wenigstens eine Beaufschlagungseinrichtung auf, welche die Kunststoffvorformlinge mit einem fließfähigen Medium zu deren Sterilisation beaufschlagt, sowie ein Gehäuse, welches den Transportpfad der Kunststoffvorformlinge wenigstens abschnittsweise umgibt. Erfindungsgemäß weist die Vorrichtung eine Erwärmungseinrichtung auf, welche wenigstens einen Wandungsabschnitt des Gehäuses, wenigstens zeitweise, erwärmt. Zusätzlich oder alternativ ist wenigstens ein Abschnitt des Gehäuses, welches den Transportpfad der Kunststoffvorformlinge umgibt, thermisch gegenüber der Umgebung der Vorrichtung isoliert.

Bevorzugt ist daher wenigstens eine Wandung, welche den Transportpfad der Kunststoffvorformlinge begrenzt, thermisch isolierend aufgebaut. So könnten beispielsweise mehrkomponentige Wandungen mit dazwischen liegenden Luftschichten zur thermischen Isolierung eingesetzt werden. Weiterhin ist es denkbar, einen geschlossenzelligen Schaumstoff aus synthetischem Kautschuk aufzukleben.

Dabei ist insbesondere diese Erwärmungseinrichtung unabhängig von der Beaufschlagungseinrichtung bzw. es wird nicht die Beaufschlagungseinrichtung auch zum Erwärmen des Gehäuses bzw. eines Abschnitts des Gehäuses verwendet.

Bei einer weiteren vorteilhaften Ausführungsform weist die Vorrichtung eine weitere Beaufschlagungseinrichtung auf, welche den Wandungsabschnitt des Gehäuses mit einem weiteren fließfähigen Medium zu dessen Erwärmung beaufschlagt. Dabei kann es sich beispielsweise um eine Zuführungseinrichtung zum Zuführen warmer Luft handeln, welche auf die jeweiligen Wände auftritt.

Es ist jedoch auch die Verwendung einer elektrisch betriebenen Erwärmungseinrichtung möglich, oder einer durch eine erwärmte Flüssigkeit gespeisten Erwärmungseinrichtung.

Die vorliegende Erfindung ist weiterhin auf eine Anlage zum Herstellen von Kunststoffbehältnissen mit einer Vorrichtung der oben beschriebenen Art gerichtet sowie einer weiteren Vorrichtung zum Behandeln von Kunststoffbehältnissen. Dabei weist die Anlage eine Verbindungsleistung auf, um ein erwärmtes Medium von dieser weiteren Vorrichtung zu der Vorrichtung zu verbringen. Bei dieser Ausgestaltung wird daher vorgeschlagen, dass eine Wärme insbesondere eine Abwärme einer weiteren Vorrichtung verwendet wird, um die Gehäusewandungen ihrerseits zu erwärmen.

Weitere Vorteile ergeben sich aus der beigefügten Zeichnung:

Dabei zeigt:

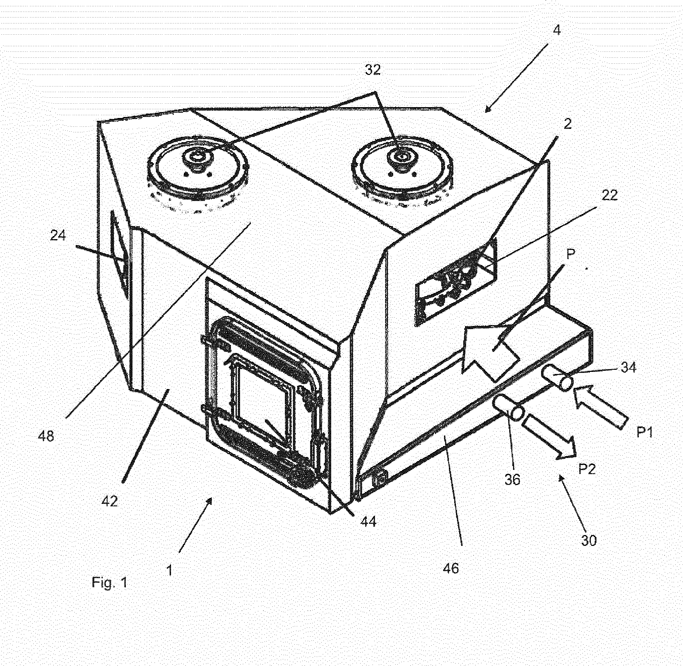

- Fig. 1: eine schematische Darstellung einer erfindungsgemäße Vorrichtung.

Figur 1 zeigt die eine schematische Darstellung einer erfindungsgemäßen Vorrichtung 1 zum Sterilisieren von Kunststoffvorformlingen. Diese Vorrichtung weist eine erste Zuführöffnung 22 auf, über welche der Vorrichtung die Behältnisse, d. h. hier die Kunststoffvorformlinge, zugeführt werden können (Pfeil P). Das Bezugszeichen 2 kennzeichnet eine Transporteinrichtung, welche die Kunsstoffvorformlinge innerhalb der Vorrichtung 1 transportiert. Diese Transporteinrichtung 2 weist dabei bevorzugt wenigstens ein Transportrad auf, an dem eine Vielzahl von Haltelementen zum Halten der Kunststoffvorformlinge angeordnet ist. Damit werden die Kunststoffvorformlinge bevorzugt auf einen kreisförmigen Transportpfad transportiert. Daneben könnten jedoch in dem Gehäuse 4 mehrere Transporteinrichtungen angeordnet sein, insbesondere mehrere Transportsterne, wobei die Kunsstoffvorformlinge beispielsweise von einem Einlaufstern an einen weiteren Transportstern übergeben und von dort weiter an einen Auslaufstern.

Das Bezugszeichen 24 kennzeichnet eine Abführöffnung bzw. einen Auslauf, um die Kunststoffvorformlinge (nunmehr sterilisiert) abzuführen. Bevorzugt ist die Zuführöffnung 22 räumlich getrennt von der Abführöffnung angeordnet.

Das Gehäuse 4 bildet in seinem Inneren einen Reinraum aus, innerhalb dessen die Kunsstoffvorformlinge sterilisiert werden. Das Bezugszeichen 32 bezieht sich auf Zuführöffnungen, um dem Gehäuse ein Prozessgas, also hier insbesondere eine Wasserstoffperoxid-Luftmischung, zuzuführen. Im Inneren des Gehäuses können Düsenelemente angeordnet sein, welche die Kunsstoffvorformlinge gezielt mit dem Sterilisationsmedium beaufschlagen. Das Innere des Gehäuses stellt damit auch einen isolierten Behandlungsbereich dar.

Das Bezugszeichen 44 bezieht sich auf ein Fenster, durch welches hindurch der Benutzer in das Innere der Vorrichtung 1 blicken kann. Dieses Fenster kann dabei bevorzugt auch geöffnet werden und kann damit eine Revisionsöffnung frei geben.

Das Bezugszeichen 42 kennzeichnet exemplarisch eine erste Wandung und das Bezugszeichen 46 eine weitere Wandung - hier einen Boden der Vorrichtung. Bei der in Figur 1 gezeigten Darstellung kann dieser Boden durch Zuführung eines Temperiermediums beheizt werden. Zu diesem Zweck ist ein Zulauf 34 vorgesehen, über welchen entlang des Pfeils P1 das Temperiermedium zugeführt wird. Das Bezugszeichen 36 kennzeichnet einen Rücklauf zum Abführen des Temperiermediums. Der Boden 46 wirkt daher auch als Wärmetauscher und wird durch diese zugeführte Luft, die beispielsweise aus einer anderen Vorrichtung (nicht gezeigt) stammen kann, zur Erwärmung eingesetzt. Das Bezugszeichen 48 kennzeichnet einen Deckel bzw. eine Deckelwandung der Vorrichtung

Weiterhin wäre es möglich, dass Antriebe für die Transporteinrichtung 2 nicht innerhalb des Gehäuses 4 bzw. des Reinraums angeordnet sind, sondern außerhalb des Gehäuses. Daneben können auch Sterilisationselemente vorgesehen sein, welche beispielsweise auch Bereiche der Transporteinrichtung selbst sterilisieren, wie beispielsweise die Greifklammern, welche die Kunststoffvorformlinge halten. Daneben wäre es jedoch auch möglich, dass eine Sterilisation derartiger Anlagenteile im Vorfeld zu einem eigentlichen Betriebsmodus durchgeführt wird.

Das Erwärmungsmedium, welches über die Zuführöffnung bzw. das Rohr 34 zugeführt und über die Abführöffnung bzw. das Rohr 36 abgeführt wird, wird damit bei der hier gezeigten Ausführungsform ausschließlich innerhalb des Wandelementes 46 geführt und gelangt damit bevorzugt nicht in den Innenraum des Gehäuses selbst. Es wäre jedoch auch möglich, dass dieses erwärmte Medium auch im Inneren des Gehäuses verteilt wird, um so auch andere Wände, wie hier die Wand 42 erwärmen zu können. So könnten beispielweise im Inneren des Gehäuses Verteileinrichtungen wie Düsenelemente vorgesehen sein, welche die Wandungen gezielt mit der warmen Luft beaufschlagen. Daneben könnten auch innerhalb der Wände Leitungskanäle für Luft angeordnet sein, um diese so zu beaufschlagen. In diesem Falle erfolgt ein Beaufschlagen der Wandungsabschnitte von innen her.

Das Bezugszeichen 30 bezieht sich auf die Erwärmungseinrichtung in ihrer Gesamtheit, welche bei der hier gezeigten Ausführungsform die beiden Leitungen 34 und 36 sowie den zu erwärmenden Wandabschnitt 46 aufweist.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Vorrichtung
- 2: Transporteinrichtung
- 4: Gehäuse
- 22: Zuführöffnung
- 24: Abführöffnung
- 30: Erwärmungseinrichtung
- 32: Zuführöffnung
- 36: Rücklauf, Abführöffnung
- 42: erste Wandung
- 44: Fenster
- 46: weitere Wandung
- P, P1, P2: Pfeile

## Patentansprüche

1. Verfahren zum Sterilisieren von Kunststoffvorformlingen, wobei die Kunststoffvorformlinge entlang eines vorbestimmten Transportpfads von einer Transporteinrichtung (2) transportiert werden und wenigstens zeitweise während dieses Transports durch Beaufschlagung mit einem fließfähigen Sterilisationsmittel sterilisiert werden, wobei die Kunststoffvorformlinge wenigstens zeitweise innerhalb eines Gehäuses (4) transportiert werden,
**dadurch gekennzeichnet, dass**
wenigstens ein Abschnitt des Gehäuses (4) wenigstens zeitweise vor und/oder während des Sterilisationsvorgangs durch eine von dem Sterilisationsmittel unabhängige Wärmequelle erwärmt wird und/oder wenigstens ein Sterilisationsbereich, in dem die Kunststoffvorformlinge sterilisiert werden, gegenüber einer Umgebung thermisch isoliert wird.

2. Verfahren nach Anspruch 1,
**dadurch gekennzeichnet, dass**
der Abschnitt mit einem weiteren erwärmten fließfähigen Medium beaufschlagt wird.

3. Verfahren nach Anspruch 2,
**dadurch gekennzeichnet, dass**
das erwärmte fließfähige Medium einer weiteren Vorrichtung zum Behandeln von Kunststoffbehältnissen entnommen wird.

4. Verfahren nach Anspruch 3,
**dadurch gekennzeichnet, dass**
die weitere Vorrichtung eine Vorrichtung zum Erwärmen von Kunststoffvorformlingen ist.

5. Verfahren nach wenigstens einem der vorangegangenen Ansprüche,
**dadurch gekennzeichnet, dass**
die Kunststoffvorformlinge innerhalb eines Reinraums sterilisiert werden.

6. Verfahren nach Anspruch 2,
**dadurch gekennzeichnet, dass**
das erwärme fließfähige Medium gereinigt wird, bevor es zu dem Abschnitt gelangt.

7. Vorrichtung (1) zum Sterilisieren von Kunststoffvorformlingen (10) mit einer Transporteinrichtung (2), welche die Kunststoffvorformlinge entlang eines vorgegebenen Transportpfads transportiert, mit wenigstens einer Beaufschlagungseinrichtung, welche die Kunststoffvorformlinge mit einem fließfähigen Medium zu deren Sterilisation beaufschlagt und mit einem Gehäuse (4), welches den Transportpfad der Kunststoffvorformlinge wenigstens abschnittsweise umgibt,
**dadurch gekennzeichnet, dass**
die Vorrichtung eine Erwärmungseinrichtung (30) aufweist, welche wenigstens einen Wandungsabschnitt (42, 46) des Gehäuses (4) wenigstens zeitweise erwärmt und/oder ein Abschnitt des Gehäuses (4), thermisch gegenüber der Umgebung der Vorrichtung isoliert ist.

8. Vorrichtung (1) nach Anspruch 7,
**dadurch gekennzeichnet, dass**
die Vorrichtung eine weitere Beaufschlagungseinrichtung (12) aufweist, welche den Wandungsabschnitt des Gehäuses mit einem weiteren fließfähigen Medium zu dessen Erwärmung beaufschlagt.

9. Anlage zum Herstellen von Kunststoffbehältnissen mit einer Vorrichtung (1) nach Anspruch 8 sowie einer weiteren Vorrichtung zum Behandeln von Kunststoffbehältnisse, wobei die Anlage eine Verbindungsleitung aufweist, um ein erwärmtes fließfähiges Medium von dieser weiteren Vorrichtung zu der Vorrichtung (1) nach Anspruch 8 zu leiten.
